# EUROPÄISCHE PATENTSCHRIFT

(11) **EP 1 784 306 B1**
(45) Veröffentlichungstag und Bekanntmachung des Hinweises auf die Patenterteilung: **04.12.2013**
(21) Anmeldenummer: 05778293.0
(22) Anmeldetag: 17.08.2005
(51) Int. Cl.: B32B 37/14, B32B 27/12, B32B 27/32, A61F 13/15

(54) **VLIES-FOLIEN-LAMINATE**
NONWOVEN/FILM LAMINATES
LAMINES DE FILMS/NON-TISSES

(30) Priorität: 02.09.2004 DE 102004042405
(43) Veröffentlichungstag der Anmeldung: 16.05.2007
(73) Patentinhaber: RKW SE, 67227 Frankenthal (DE)
(72) Erfinder: BÖRMANN, Ludwig, 83547 Babensham (DE); SCHREINER, Günter, 84518 Garching/Alz (DE)
(74) Vertreter: Wagner, Jutta
(86) Internationale Anmeldenummer: PCT/EP2005/008903
(87) Internationale Veröffentlichungsnummer: WO 2006/024394

(56) Entgegenhaltungen:
- EP-A- 0 650 828
- EP-A- 0 841 156
- WO-A-94/20298
- US-A- 5 879 614

## Beschreibung

Die Erfindung betrifft verbesserte Vlies-Folien Laminate sowie ein Verfahren zu ihrer Herstellung.

Kunststofffolien sind als wasserdichtes und gewünschtenfalls gleichzeitig atmungsaktives Material in vielen Bereichen der Technik und des täglichen Lebens nicht mehr wegzudenken. Ein weiter Anwendungsbereich betrifft Hygieneartikel, wie z.B. Windeln.

Während bekannte Folien die Anforderungen im Hinblick auf Dichtigkeit, Leichtigkeit und Atmungsaktivität gut erfüllen, sind die Stabilität und vor allem die als "Griff" bezeichnete Oberflächenbeschaffenheit nicht optimal. Insbesondere atmungsaktive Folien reißen leicht, der Einsatz dickerer und damit stabilerer Folien lässt die Kosten steigen. Im Hinblick auf den Griff wird die glatte, glänzende Oberfläche von Kunststofffolien als unangenehm empfunden. Besonders die innere, an der Haut anliegende Oberfläche von Hygieneprodukten und auch die Außenseite soll sich weich und möglichst textil-ähnlich anfühlen. Glatte Folien vermitteln den Eindruck an der Haut zu kleben, selbst wenn sie atmungsaktiv sind.

Ein weiteres Problem ist die als Rascheln bezeichnete Geräuschentwicklung, die speziell bei dünnen Folien in Hygieneartikeln bei Bewegungen des Trägers/der Trägerin auftritt. Ein Rascheln ist so weit wie irgend möglich zu vermeiden, da ansonsten die Akzeptanz des Hygieneproduktes leidet.

Zur Überwindung der genannten Probleme sind unzählige Vorschläge zur Veränderung der Folien an sich gemacht worden und auch zum Einsatz von Laminaten aus Folien mit Geweben oder Vliesen.

So wird beispielsweise in DE 195 38 049 ein Verfahren zur Herstellung einer Folienbahn beschrieben, die einerseits eine verbesserte Querelastizität und Durchstoßfestigkeit und andererseits eine verbesserte Weichheit und vermindertes Rascheln aufweisen. Dabei wird eine Ausgangsfolienbahn aus thermoplastischem Polymermaterial mittels eines oder mehrerer Heizzylinder bis zum schmelzflüssigen Zustand über den Kristallitschmelzpunkt des Polymermaterials erwärmt und danach durch einen gekühlten Walzenspalt geführt.

Weit verbreitet sind auch Laminate aus Vlies bzw. Gewebe und Folien, da diese die Wasserdichtigkeit der Folie mit der textilähnlichen Oberfläche von Gewebe bzw. Vlies verbinden. Überwiegend werden Vliese eingesetzt. Die Laminate bestehen im einfachsten Fall aus einer Folie und einem Vlies, welche auf verschiedene Weise miteinander verbunden sein können. Die gängigsten Verfahren sind Thermobonding, Kleben und Direktextrusion (Castverfahren).

Beim Thermobonding wird mittels einer Prägewalze (= gravierte Stahlwalze) zumeist mit einer glatten Stahlwalze als 2. Walze punktuell das Material von Folie und/oder Vlies durch hohe Temperatur und Druck angeschmolzen, wobei sich eine Verbindung der beiden Materialbahnen ergibt. Das Verfahren hat den Nachteil, dass durch die Bedingungen beim Verbinden die Folie beschädigt werden und dabei die Flüssigkeitsdichtigkeit verlieren kann, sog. Pinholing. Auch wird nur eine punktuelle Verbindung erreicht, was sich nachteilig auf die Verbundfestigkeit auswirkt.

Ein Beispiel ist in der US 5,837,352 gegeben, welche Laminate aus Folie und Vlies beschreibt, die unter anderem durch Thermobonding oder auch durch Ultraschallverfahren verbunden sein können.

Das Kleben erreicht eine vollflächige Verbindung ist aber bei atmungsaktiven Folien mit einer Verschlechterung der Atmungsaktivität verbunden. Zudem verursacht der Klebstoff zusätzliche Kosten und Klebstoffe stehen teilweise in Verdacht, gesundheitlich nicht unbedenklich zu sein. Wenn zum Erhalt der Atmungsaktivität keine flächige sondern eine punktuelle Verklebung gewählt wird, leidet wiederum die Verbundfestigkeit.

Als Variante zum Kleben kann das Vorsehen von Folien und/oder Vlieslagen bzw. das Einbringen von Zusätzen in Folie und/oder Vlies angesehen werden, welche beim Thermobonding zu einer Verklebung bei wesentlich niedrigeren Temperaturen führen. Ein Beispiel ist in der US 5,695,868 beschrieben, bei der entweder in Folie oder Vlies oder in beiden eine als "bonding agent" bezeichnete Komponente enthalten ist. Diese Komponente ermöglicht ein Thermobonding unterhalb des Schmelzpunktes von Folie und Vlies, so dass die Atmungsaktivität der Folie erhalten bleibt. Die Verbindung bleibt aber punktuell.

Die Direktextrusion ist für nicht atmungsaktive Laminate ein preiswertes Verfahren, das zu einer sicheren Verbundfestigkeit führt aber eine geringe Weichheit und ein hohes Risiko für Pinholing bedingt. Sofern atmungsaktive Laminate gewünscht sind, kann die Atmungsaktivität nur in einem zweiten Schritt durch Nachbearbeitung des Verbundes erreicht werden. Dazu sind entweder in der Folie Füllstoffe enthalten, an denen sich bei einem Recken des Laminates Poren bilden oder das Laminat wird durch Vernadeln mit Poren versehen.

Als Beispiel sei die US 5,865,926 genannt, in der eine Folie auf eine Vliesbahn extrudiert wird und anschließend der Verbund mittels Oberflächenstrukturierter Walzen gereckt wird (Ringrolling), um den Verbund atmungsaktiv zu machen.

Zuletzt finden auch Verfahren Anwendung, bei denen Vliese mit einer Beschichtung versehen werden, welche die gewünschte Dichtigkeit gegen Flüssigkeiten bei gleichzeitiger Durchlässigkeit für Wasserdampf bewirken. Ein Beispiel hierfür ist die US 5,879,341.

Keines der bekannten Verfahren ist hinsichtlich aller Forderungen optimal. Es besteht daher ein ständiger Bedarf an verbesserten Laminaten und verbesserten Verfahren zur Herstellung von Laminaten.

Überraschend wurde nun gefunden, dass sich hinsichtlich Atmungsaktivität, Weichheit und Raschelverhalten sehr gute Laminate herstellen lassen, indem eine Folie bis zum schmelzeflüssigen Zustand erwärmt und bei dieser Temperatur mit einem Vlies zusammengeführt und der Verbund durch einen gekühlten Walzenspalt geführt wird.

Die obigen Aufgaben werden daher gelöst durch ein Verfahren zur Herstellung eines Laminates aus einer Ausgangsfolienbahn und einer Ausgangsvliesbahn, wobei die Ausgangsfolienbahn aus einem thermoplastischen Polymermaterial zusammen mit der Ausgangsvliesbahn, deren Schmelzpunkt oberhalb dem Kristallitschmelzpunkt des Polymermaterials liegt, über den Kristallitschmelzpunkt des Polymermaterials hinaus erwärmt und das Laminat durch einen gekühlten Walzenspalt geführt wird, sowie durch nach diesem Verfahren erhältliche Laminate.

Überraschenderweise verbindet sich das schmelzeflüssige Polymermaterial der Folienbahn mit der nicht schmelzeflüssigen Vliesbahn. Fixiert wird dieser Verbund im anschließenden gekühlten Walzenspalt.

Die Ausgangsfolienbahn wird in an sich bekannter Weise hergestellt, z.B. blasextrudiert. Als Materialien für die Folie kommen im Prinzip alle thermoplastischen Polymere in Betracht. Hierzu ist eine Vielzahl von Handelsprodukten am Mark erhältlich. Vorzugsweise werden LDPE (Low Density Polyethylen), LLDPE (Linear Low Density Polyethylen), MDPE (Medium Density Polyethylen), HDPE (High Density Polyethylen) und verschiedene PP (Polypropylen) sowie Copolymerisate von Ethylen oder Propylen mit anderen Comonomeren eingesetzt. Diese Polymere werden entweder in reiner Form oder als Polymergemische verwendet. Übliche Rezepturen für Hygienefolien sind z. B Gemische aus 10 bis 90 Gew.-% LDPE, 10 bis 90 Gew.-% LLDPE und 0 bis 50% MDPE, zum Beispiel ein Gemisch aus 80% LDPE, 20% LLDPE und anforderungsgerechten Pigmentierungen. Handelsübliche Polymere für Hygienefolien besitzen die nachfolgend angegebenen Schmelzbereiche bzw. Kristallitschmelzpunkte:
LDPE = 112 bis 114 °C,
LLDPE = 119 bis 125 °C
MDPE = 125 bis 128 °C.

Üblicherweise werden Hygienefolien eingefärbt, z. B. weiß mit Titandioxid. Hinzu kommen übliche Zusatzstoffe und Verarbeitungshilfsmittel, die teilweise Betriebsgeheimnis der Verarbeiter sind.

Weiterhin sind Ethylenvinylacetat (EVA), Ethylenacrylat (EEA), Ethylenethylacrylat (EEA) Ethylenacrylsäure (EAA), Ethylenmethylacrylat (EMA), Ethylenbutylacrylat (EBA), Polyester (PET), Polyamid (PA) z.B. Nylon, Ethylenvinylalkohole (EVOH), Polystyrol (PS), Polyurethan (PU) und thermoplastische Olefinelastomere geeignet.

Als Material der Ausgangsfolienbahn sind Polyolefine wie z.B. LDPE, LLDPE und PP bevorzugt. Besonders bevorzugt sind Mischungen dieser Polymere, wie beispielsweise Mischungen aus LDPE und LLDPE, Mischungen aus LDPE oder LLDPE und PP oder Mischungen von PE oder PP verschiedener Schmelzpunkte.

Auch die Ausgangsvliesbahn wird in an sich bekannter Weise hergestellt. Geeignet sind alle Vliesstoffe, die zumindest eine Rezepturkomponente auf Basis eines thermoplastischen Polymers enthalten. Die Vliese können Fasern aus PE, PP, PET, Rayon, Zellulose, PA und Mischungen dieser Fasern enthalten. Auch Bi- oder Multikomponentenfasern sind brauchbar. Besonders bevorzugt werden z.B. Vliese aus Spinn- oder Stapelfaser auf Basis von PP, PE oder PET, sowie Vliese aus Mischungen von PP und PE oder Mischungen von PET und PP oder PE.

Das erfindungsgemäße Thermolaminierverfahren funktioniert generell bei allen thermoplastischen Rezepturen, wobei die Schmelzpunkte und Rohstoffe ebenso wie beim Thermobonding aufeinander abgestimmt sein müssen. Generell müssen die zu laminierenden Bahnen zumindest in einer Rezepturkomponente eine ähnliche Morphologie aufweisen damit die Basis für eine ausreichende Verbundhaftung über die Temperatur gegeben ist.

Die Anzahl der zu laminierenden Bahnen ist nicht begrenzt, es muss nur für die erforderliche Erwärmung der Bahnen z.B. über einen Heizzylinder bis zum gekühlten Walzenspalt gesorgt werden. Es können nicht nur Vliese mit Folien laminiert werden sondern jede erdenkliche Kombination (z.B. Vlies/Vlies; Vlies/Folie/Vlies; Folie/Folie; etc.).

Die Ausgangsbahnen können durch jedes bekannte Verfahren hergestellt worden sein müssen jedoch thermoplastische Anteile beinhalten. Wichtig ist, dass die Materialien von Folie und Vlies aufeinander abgestimmt werden, indem einerseits die Kristallitschmelzpunkte weit genug auseinander liegen und andererseits die Materialien soweit miteinander verträglich sind, dass eine Verbindung ermöglicht ist. Geeignete Materialkombinationen sind dem Fachmann bekannt bzw. können anhand einiger weniger orientierender Versuche ermittelt werden.

Der Unterschied in der Kristallitschmelztemperatur der Ausgangsfolienbahn bzw. der niedrigschmelzenden Komponente der Ausgangsfolienbahn sollte mindestens etwa 5 °C, vorzugsweise mindestens etwa 10°C und insbesondere mindestens etwa 20 °C unterhalb der Schmelztemperatur der Ausgangsvliesbahn bzw. unterhalb der Schmelztemperatur der hochschmelzenden Komponente der Ausgangsvliesbahn liegen.

Zur Verbesserung der Verträglichkeit kann in der Ausgangsfolienbahn oder der Ausgangsvliesbahn oder in beiden eine niedrig schmelzende Komponente enthalten sein. Hierbei ist zu gewährleisten, dass zumindest eine Komponente der Ausgangsvliesbahn einen Schmelzpunkt oberhalb der Kristallitschmelztemperatur der Ausgangsfolienbahn bzw. der niedriger schmelzenden Komponente in der Ausgangsfolienbahn aufweist.

Zur Verbesserung der Materialverträglichkeit ist es auch möglich, zwei- oder mehrlagige Vliese einzusetzen, bei denen die im Laminat mit der Ausgangsfolienbahn in Kontakt befindliche Lage im Vergleich zu der/den weiteren Lagen aus einem niedriger schmelzenden Material besteht bzw. ein niedriger schmelzendes Material enthält.

Die Ausgangsfolienbahn wird erfindungsgemäß gemeinsam mit der Ausgangsvliesbahn über einen vorzugsweise antihaft beschichten Heizzylinder erwärmt und anschließend durch einen gekühlten Walzenspalt geführt. Es kann natürlich auch mit mehreren Heizzylindern oder anderen Erwärmungsmethoden wie z.B. Infrarotstrahler gearbeitet werden, im folgenden wird der Übersichtlichkeit halber die Erfindung jedoch nur anhand der Erwärmung mit einem Heizzylinder beschrieben.

In einer bevorzugten Ausführungsform steht die Ausgangsvliesbahn im direkten Kontakt mit der Heizzylinderoberfläche. Darüber wird die Ausgangsfolienbahn mitgeführt. Die Temperatur des Heizzylinders wird so gewählt, dass über die Umschlingungsstrecke des Heizzylinders die Ausgangsfolienbahn in den schmelzeflüssigen Zustand erwärmt wird, diese Temperatur aber für die mitgeführte Ausgangsvliesbahn noch nicht den schmelzeflüssigen Zustand auslöst, d.h. unterhalb des Kristallitschmelzpunkts der Ausgangsvliesbahn liegt. Da die noch nicht im schmelzeflüssigen Zustand befindliche Vliesbahn auf dem Heizzylinder aufliegt ist ein leichtes und sehr prozessstabiles Ablösen der darüberliegenden schmelzeflüssigen Folienbahn gewährleistet.

Es ist aber auch möglich, das Verfahren mit direktem Kontakt der Folienbahn zu einem antihaft beschichteten Heizzylinder und einer darüberliegenden Vliesbahn durchzuführen.

Im anschließenden gekühlten Walzenspalt wird das Laminat auf Temperaturen unterhalb des Kristallitschmelzpunkts der Folienbahn gekühlt. Der gekühlte Walzenspalt besteht bevorzugt aus einer Stahlwalze und einer im Gegendruck arbeitenden Gummiwalze. Die Stahlwalze wird vorzugsweise mit einer textilähnlichen Gravur ausgestattet, die das textile Erscheinungsbild der Oberfläche des Laminates weiter verstärkt. Die vorzugsweise verwendete Prägestruktur der Stahlwalze reduziert den Glanzgrad des Laminats.

Im Gegensatz zu bekannten Thermobonding Laminierverfahren, wobei durch zwei beheizte Stahlwalzen (erhabene Gravurwalze und glatte Gegendruckwalze) eine Folienbahn und Vliesbahn geführt wird und mittels Temperatur und sehr hoher Drücke der Verbund nur punktuell geschaffen wird, arbeitet das erfindungsgemäße Thermolaminierverfahren mit einer vollflächigen Laminierung. Dies bietet, ähnlich den bekannten Kleberlaminierverfahren, den Vorteil, dass durch geringe Drücke im Laminierprozeß (Erwärmung drucklos, Laminierschritt im gekühlten Walzenspalt vollflächig und mit weicher Gummiwalze als Gegendruckwalze) sehr weiche Laminate (ähnlich Kleber Laminaten) ohne Verwendung von Klebern geschaffen werden und andererseits die bei den Thermobondinglaminaten bestehende Gefahr von Materialschädigungen (Löcher, Pinholes) ausgeschlossen ist.

Die Steuerung der Verbundhaftung zwischen Vlies und Folie kann sehr leicht über den Grad der Erwärmung eingestellt werden. Eine höhere Temperatur der Heizzylinderoberfläche bedeutet gleichzeitig höhere Verbundwerte zwischen Vlies und Folie. Das Prozessfenster der Erwärmung ist bei der minimalen Temperatur durch den zwingend erforderlichen schmelzeflüssigen Zustand der für die Verbundhaftung verantwortlichen Rohstoffkomponente gegeben. Nach oben wird die Erwärmung durch den Kristallitschmelzpunkt der Vliesbahn begrenzt. Wird in den Kristallitschmelzpunkt der Vliesbahn hinein erwärmt, entstehen zwar Laminate mit einem unzerstörbarem Verbund zwischen Vlies- und Folienbahn, allerdings geht die gute Weichheit des Laminates verloren und es entsteht die Gefahr von Löchern ähnlich wie bei der Direktextrusion oder dem Thermobonding.

Das erfindungsgemäße Thermolaminierverfahren bietet gegenüber den bekannten Kleberlaminierverfahren den Vorteil, dass eine gute Weichheit der Produkte ohne Verwendung von Kleber erreicht wird. Weiterhin können ohne größere Anlagenmodifikationen Trilaminate (Vlies/Folie/Vlies) hergestellt werden. In diesem Falle wird nur eine zweite Vliesbahn über den beiden Ausgangsbahnen mitgeführt und auch mittels Heizzylinder(n) erwärmt.

Die Vorteile des Thermolaminierverfahrens zum klassischen Thermobondingverfahren liegen in der erhöhten Weichheit der hergestellten Laminate und der Vermeidung von Materialschädigungen durch hohe Drücke und Temperaturen. Damit haben diese Laminate nicht die Gefahr von Löchern oder Mikrolöcher ("Pinholes"), die beispielsweise bei Verwendung als Sperrschicht (Backsheets) in Hygieneprodukten einen erheblichen Mangel darstellen (undichte Windeln).

Im Vergleich zu Laminaten, die nach dem bekannten Verfahren der Direktextrusion hergestellt sind, zeigen die erfindungsgemäßen Vlies-Folienkombinationen höhere Weichheit und die Gefahr von Fehlstellen (Löcher, Pinholes) ist deutlich reduziert. Bei der Direktextrusion wird der Schmelzefilm direkt nach der Breitschlitzdüse und bei Extrusionstemperatur auf das Vlies aufgetragen. In einem nachgeschalteten gekühltem Walzenspalt wird der Verbund Vlies-/Folienbahn hergestellt. Bedingt durch die notwendigen hohen Extrusionstemperaturen (z.B. ca. 230°C bei LDPE/LLDPE/PP Mischungen - das entspricht etwa 70°C oberhalb des Kristallitschmelzpunktes der höchstschmelzenden Rezepturkomponente) kommt die schmelzflüssige Folienbahn sehr dünnflüssig auf die Vliesbahn zu liegen. Dadurch durchdringt die dünnflüssige Folienbahn in Verbindung mit dem nachgeschalteten gekühlten druckangestellten Walzenspalt weitgehendst die Vliesbahn. Dies führt zu einer Verhärtung des Laminates und die Vliesfilamente durchstoßen die Folienbahn, was wiederum der Auslöser für Löcher/Pinholes im Endprodukt ist.

Im Gegensatz dazu kann erfindungsgemäß die Viskosität der schmelzeflüssigen Folienbahn sehr genau über den Heizzylinder eingestellt werden. Durch die Entkopplung des Extrusions- und Laminiervorgangs können die hohen Extrusionstemperaturen auf diesen Vorgang begrenzt werden und der Laminiervorgang mit wesentlich niedrigeren Temperaturen arbeiten. Damit kann erfindungsgemäß im Gegensatz zum Direktextrusionsverfahren - bei identischen Rezepten - bereits ab Erreichen des Kristallitschmelzpunktes der für das Laminierverhalten verantwortlichen Rohstoffkomponente in Folienbahn mit den üblich geforderten Verbundwerten für Hygieneprodukte (>0,10 N/cm) laminiert werden.

Ein weiterer Vorteil der vorliegenden Erfindung im Vergleich zur Direktextrusionslaminierung besteht in den Möglichkeiten der Bedruckung der erfindungsgemäß hergestellten Laminate. Erfindungsgemäß kann die Folie nach dem Extrusionsvorgang und vor dem Laminiervorgang bedruckt werden. Dies ermöglicht den Druck auf die Folienseite die im späteren Laminat mit dem Vlies abgedeckt wird. Damit erreicht man eine sehr gute Druckqualität, weil auf die glatte Folienbahn gedruckt werden kann. Das direkt darüberliegende Vlies im fertigen Laminat beeinflusst das Druckbild nur unerheblich, verhindert aber den Druckfarbenabrieb im Endprodukt. Beispielsweise kann bei Verwendung der erfindungsgemäß bedruckten Laminate als Backsheets für Babywindeln keine Verschmutzung des Umfeldes durch Abrieb der Druckfarbe auftreten, da diese durch das Vlies abgedeckt ist.

Bei dieser Ausführungsform ergibt sich im Hinblick auf atmungsaktive Laminate ein weiterer Vorteil, indem die Reckung zur Schaffung der Atmungsaktivität bei füllstoffhaltigen Folien bevorzugt ebenfalls vor dem Bedrucken und dem Laminiervorgang erfolgt. Hierdurch wird eine Verzerrung der Druckmotive beim Recken vermieden.

Im Falle der Direktextrusion - die schmelzeflüssige Folienbahn wird direkt nach der Breitschlitzdüse mit der Vliesbahn verbunden - besteht keine Möglichkeit auf die Folienbahn zu Drucken und anschließend mit der Vliesbahn abzudecken. Bei diesem Verfahren kann entweder direkt auf die Vliesseite (= Außenseite der verschiedenen Endprodukte (z.B. Windeln)) gedruckt werden, was durch die vielen einzelnen Vliesfilamente nur sehr eingeschränkte Druckqualitäten zulässt und im Endprodukt die Gefahr von Druckfarbenabrieb ergibt. Auch ein Druck auf die folienzugewandte Seite vor dem Aufbringen der Folie ist denkbar, aber die Druckqualität bleibt schlecht und ein Drucken auf Vlies erfordert große Mengen Druckfarbe. Eine weitere Möglichkeit des Druckens von Direktextrusionslaminaten besteht auf die Folienseite im sogenannten Konterdruckverfahren. Dabei sieht man im Endprodukt durch die Vlies- und Folienlage hindurch auf das Druckmotiv. Besonders bei stark eingefärbten opaken Folien (z.B. bei kreidegefüllten atmungsaktiven Folien) ist das Druckmotiv dann nur sehr eingeschränkt bzw. überhaupt nicht zu erkennen.

Selbstverständlich kann sich das erfindungsgemäße Verfahren auch unmittelbar an die Herstellung der Ausgangsfolienbahn anschließen, indem z.B. über eine Breitschlitzanlage eine Folienbahn extrudiert und mittels Prägewerk, Chill-Roll System oder auch nur Kühlwalze(n) gekühlt wird. Nachgeschaltet befindet sich dann in dieser Anlage einen oder mehrere erfindungsgemäße(n) Heizzylinder mit anschließendem gekühltem Walzenspalt. Gewünschtenfalls wird ein Druckwerk vor den(die) erfindungsgemäße(n) Heizzylinder geschaltet.

In einer weiteren bevorzugten Ausführungsform wird nach dem Laminiervorgang die Vlies-Folienkombination noch einem Ringrolling quer zur Bahn unterzogen. Dieses Recken in Querrichtung (CD) reduziert einerseits das Flächengewicht des Laminates, verbreitert die Bahn in Querrichtung und erhöht die Weichheit des Endproduktes. Diese beschriebenen Eigenschaftsveränderungen können leicht durch die verwendete Geometrie und den Eingriffsgrad der Ringrollingwalzen beeinflusst werden. Selbstverständlich können die Laminate auch einem Ringrolling in Maschinenrichtung (MD) oder in Quer- und Maschinenrichtung (CD+MD) unterzogen werden. Durch die gute Steuerungsmöglichkeit der Eindringtiefe der Folienbahn in die Vliesbahn über den Heizzylinder ist Lochbildung bzw. Pinholes beim Reckvorgang leicht zu vermeiden.

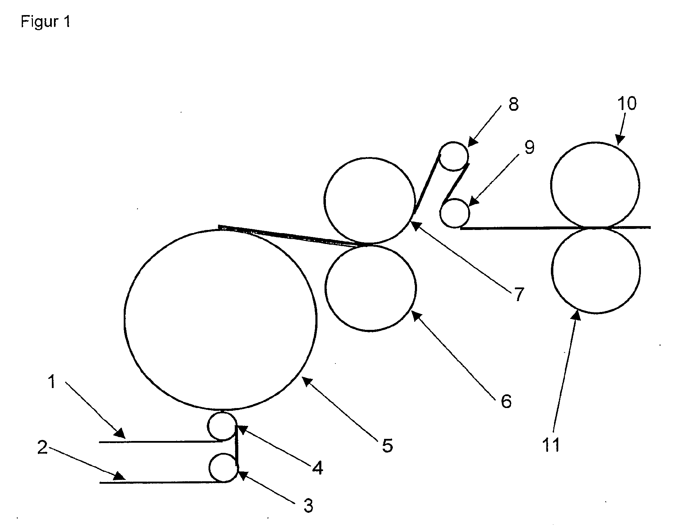

In Figur 1 ist das erfindungsgemäße Thermolaminierverfahren schematisch dargestellt. Eine Ausgangsvliesbahn 2 wird über die Umlenkwalze 3 und eine Ausgangsfolienbahn 1 über die Umlenk- und Anpresswalze 4 auf einen Heizzylinder 5 geführt. Dort werden beide Bahnen miteinander auf eine Temperatur oberhalb des Kristallitschmelzpunktes der Ausgangsfolienbahn und unterhalb des Kristallitschmelzpunktes der Ausgangsvliesbahn erwärmt. Die Vliesbahn liegt dabei an dem Zylinder 5 an. Anschließend wird der auf dem Heizzylinder 5 gebildete Verbund in dem Prägewerk aus Prägewalze 6 und Gummiwalze 7 fixiert und gekühlt. Über die Umlenkwalzen 8 und 9 wird der Verbund einer Reckung zugeführt, die durch die Ringrollingwalzen 10 und 11 in Querrichtung erfolgt. Das fertige Laminat kann dann in an sich bekannter Weise weiter verarbeitet werden.

Die folgenden Beispiele sollen die Erfindung veranschaulichen, ohne sie jedoch darauf zu beschränken.

### Beispiel 1: Vlies-Folienlaminat (nicht atmungsaktiv)

Eine Ausgangsfolienbahn aus 30% Polypropylen (Schmelzpunkt 137 - 143 °C), 60% LDPE und 10% LLDPE wird in 14 g/m² blasextrudiert. Die Ausgangsfolienbahn wird anschließend gemeinsam mit einer auf Polypropylen basierenden Ausgangsvliesbahn (14 g/m²) einer Vorrichtung, wie sie in Figur 1 schematisch dargestellt ist, zugeführt. Das Polypropylen der Folienbahn unterscheidet sich durch einen um ca. 20°C geringeren DSC-Kristallitschmelzpunkt von dem Polypropylen der Ausgangsvliesbahn. Die Ausgangsvliesbahn steht im direkten Kontakt mit der Heizzylinderoberfläche. Darüber wird die Ausgangsfolienbahn mitgeführt. Die Temperatur des Heizzylinders wird so gewählt, dass über die Umschlingungsstrecke des Heizzylinders die Ausgangsfolienbahn mit einer Temperatur von 137 bis 143 °C in den schmelzeflüssigen Zustand erwärmt wird. Bei dieser Temperatur erreicht die mitgeführte Ausgangsvliesbahn noch nicht den schmelzeflüssigen Zustand. Im anschließenden gekühlten Walzenspalt wird das Laminat unterhalb den Kristallitschmelzpunkt der Folienbahn abgekühlt.

Das Laminat zeigte die für Hygieneprodukte geforderten Verbundwerte > 0,10 N/cm.

### Beispiel 2: Vlies-Folienlaminat (nicht atmungsaktiv)

Eine Ausgangsfolienbahn aus 70% LDPE (Schmelzpunkt 108-113°C) und 30% LLDPE (117-124°C) wird in 14 g/m² blasextrudiert. Die Ausgangsfolienbahn wird anschließend gemeinsam mit einer auf Polyethylen basierenden Ausgangsvliesbahn (14 g/m², Schmelzpunkt 131-135°C) einer Vorrichtung, wie sie in Figur 1 schematisch dargestellt ist, zugeführt. Das LDPE der Folienbahn unterscheidet sich durch einen um ca. 20°C geringeren DSC-Kristallitschmelzpunkt von dem Polyethylen der Ausgangsvliesbahn. Die Ausgangsvliesbahn steht im direkten Kontakt mit der Heizzylinderoberfläche. Darüber wird die Ausgangsfolienbahn mitgeführt. Die Temperatur des Heizzylinders wird so gewählt, dass über die Umschlingungsstrecke des Heizzylinders die Ausgangsfolienbahn mit einer Temperatur von 114 bis 125°C partiell (über LDPE Schmelzpunkt) oder ganz (über LDPE und LLDPE Schmelzpunkt) in den schmelzeflüssigen Zustand erwärmt wird. Bei dieser Temperatur erreicht die mitgeführte Ausgangsvliesbahn noch nicht den schmelzeflüssigen Zustand. Im anschließenden gekühlten Walzenspalt wird das Laminat unterhalb den Kristallitschmelzpunkt der Folienbahn abgekühlt. Für einen ausreichenden Verbund ist bereits der schmelzeflüssige Zustand der LDPE-Rezepturkomponente ausreichend.

Das Laminat zeigte die für Hygieneprodukte geforderten Verbundwerte > 0,10 N/cm.

### Beispiel 3: Vlies-Folienlaminat (atmungsaktiv)

Eine Ausgangsfolienbahn (Precursor-Film) wird blasextrudiert. Die Rezeptur der Folienbahn besteht aus 70% Polypropylen-Compound (Schmelzpunkt 137 - 143 °C) und 30% LLDPE-Compound (Schmelzpunkt 117 - 124°C), wobei die Compounds jeweils aus einer Abmischung Rohstoff plus 60% CaCO₃ (Kreide) bestehen. Die Ausgangsfolienbahn wird anschließend gemeinsam mit einer auf Polypropylen basierenden Ausgangsvliesbahn (14 g/m²) einer Vorrichtung, wie sie in Figur 1 schematisch dargestellt ist zugeführt. Das in der Folienbahn anwesende Polypropylen unterscheidet sich durch einen um ca. 20°C geringeren DSC-Kristallitschmelzpunkt von dem Polypropylen der Ausgangsvliesbahn. Die beiden Bahnen werden gemeinsam über einen antihaft beschichten Heizzylinder erwärmt und anschließend durch einen gekühlten Walzenspalt (Prägewalze plus als Gegendruck arbeitende Gummiwalze) geführt. Die Ausgangsvliesbahn steht im direkten Kontakt mit der Heizzylinderoberfläche. Darüber wird die Ausgangsfolienbahn mitgeführt. Die Temperatur des Heizzylinders wird so gewählt, dass über die Umschlingungsstrecke des Heizzylinders die Ausgangsfolienbahn auf 137 - 143°C in den schmelzeflüssigen Zustand erwärmt wird bei dieser Temperatur aber die mitgeführte Ausgangsvliesbahn noch nicht den schmelzeflüssigen Zustand erreicht. Im anschließenden gekühlten Walzenspalt wird das Laminat unterhalb den Kristallitschmelzpunkt der Folienbahn abgekühlt.

Im vorliegenden Ausführungsbeispiel wird nach dem Laminiervorgang die Vlies-Folienkombination noch einem Ringrolling quer zur Materialbahn unterzogen. Bei diesem Reckvorgang wird die Atmungsaktivität erzeugt. D.h. es entstehen um die Kreidekörner (mittlere Partikelgröße 0.8 - 3.0µm) feine Poren zur Erreichung der Atmungsaktivität mit einer maximal erlaubten Größe von ca. 1µm zur Beibehaltung der Flüssigkeitsdichtigkeit. Die Messung nach ASTM E 398 (38°C, 90% relative Luftfeuchte, Messgerät LYSSY L 80 - 5000 Lyssy AG, CH) ergab eine Wasserdampfdurchlässigkeit von 2200 - 2500 g/m² in 24h.

Je nach Größe der verwendeten Kreidekörner und Eindringtiefe des Ringrolling lassen sich Wasserdampfdurchlässigkeiten von 500 - 3500 g/m² in 24h erreichen.

### Beispiel 4: Vlies-Folienlaminat (hoch atmungsaktiv)

Zunächst wird eine Ausgangsfolienbahn (Precursor-Film) blasextrudiert. Die Rezeptur der Folienbahn besteht aus 70% niedrigschmelzendem Polypropylen-Compound (Schmelzpunkt ca. 130 °C) und 30% hochschmelzendem Polypropylen-Compound(Schmelzpunkt 158 - 164°C). Die Compounds bestehen jeweils aus einer Abmischung Rohstoff plus 55% CaCO₃ (Kreide).

Nach der Blasextrusion der sog. Precursorfolie wird die Folie über eine monoaxiale MDO-Reckanlage in Maschinenrichtung gereckt. Dabei wird 100% der Folienbahn mit einem Reckgrad von 1:1,5 bis 1:4,0 in Maschinenrichtung gereckt und damit die Atmungsaktivität erzeugt. Im Gegensatz zum partiellen Recken beim Ringrolling können beim MDO-Recken über hohe Reckgrade und der gesamten zur Verfügung stehenden Folienbahnfläche fürs Recken sehr hohe Atmungsaktivitäten erreicht werden ohne dass das Problem von Löchern oder Pinholes und damit feuchtigkeitsundichte Folienbahnen auftaucht.

Im Anschluss an den Reckvorgang kann die atmungsaktive Folienbahn gewünschtenfalls sehr leicht mit üblichen Verfahren bedruckt werden. Die glatte Folienoberfläche bietet die Basis für sehr exakte Druckbilder auf der Folienbahn und die Druckmotive bleiben durch den vorgeschalteten Reckvorgang unverzerrt erhalten. Beispielsweise werden atmungsaktive Direktextrusionslaminate (Vlies-Folienlaminate) erst nach dem Druckvorgang auf die Vliesbahn und Laminiervorgang gereckt. Dabei tritt eine Verzerrung der Druckmotive über diesen nachgeschalteten Reckvorgang auf.

Die atmungsaktive Folienbahn wird anschließend gemeinsam mit einer auf Polypropylen basierenden Ausgangsvliesbahn (14 g/m²) über einen antihaft beschichten Heizzylinder erwärmt und anschließend durch einen gekühlten Walzenspalt geführt. Das in der Folienbahn anwesende Polypropylen unterscheidet sich durch einen um ca. 20°C geringeren DSC-Kristallitschmelzpunkt von dem hochschmelzenden Polypropylen der Ausgangsvliesbahn. Das Rezept der dreilagigen Ausgangsvliesbahn besteht in zwei Vlieslagen aus einem hochschmelzenden Polypropylen (Kristallitschmelzpunkt ca. 150 - 165°C). Die dritte Vlieslage (=Außenlage = Laminierseite zur Folie) wurde ähnlich der Folienbahn mit einem Polypropylen mit Schmelzpunkt ca. 130 °C ausgeführt.

Die Ausgangsvliesbahn steht im direkten Kontakt mit der Heizzylinderoberfläche wobei die Vlieslage mit reduziertem Schmelzpunkt zur Ausgangsfolienbahn zeigt. Darüber wird die Ausgangsfolienbahn mitgeführt. Die Temperatur des Heizzylinders wird so gewählt, dass über die Umschlingungsstrecke des Heizzylinders die Ausgangsfolienbahn partiell in den schmelzeflüssigen Zustand des niedrigschmelzenden PP-Compounds erwärmt wird, diese Temperatur von 130 bis 140 °C aber für die mitgeführte Ausgangsvliesbahn noch nicht den schmelzeflüssigen Zustand der beiden hochschmelzenden PP-Vlieslagen erreicht. Die dritte niedrigschmelzende PP-Vlieslage wird dabei bis in den Kristallitschmelzpunkt erwärmt. Diese Vlieslage steht im direkten Kontakt mit der Ausgangsfolienbahn und unterstützt über den schmelzeflüssigen Zustand einen ausreichenden Verbund (>0,10 N/cm). Im anschließenden gekühlten Walzenspalt wird das Laminat unterhalb Kristallitschmelzpunkt aller Rezepturkomponenten der Folien- und Vliesbahn gekühlt. Es wurde eine Wasserdampfdurchlässigkeit von 2000 - 3500 g/m² in 24h gemessen.

Wie in Beispiel 2 lassen sich durch Variation der Parameter Korngrößen des Füllstoffs und Eindringtiefe beim Ringrolling die Wasserdampfdurchlässigkeiten beeinflussen. Werte im Bereich von 500 - 5000 g/m² in 24h sind möglich.

Ein zusätzliches Ringrolling in CD erhöht die Weichheit und Wasserdampfdurchlässigkeit noch weiter.

## Patentansprüche

1. Verfahren zur Herstellung von Vlies-Folien-Laminaten, welche als Sperrschicht für Hygieneartikel verwendbar sind, aus einer Ausgangsfolienbahn aus einem thermoplastischen Polymermaterial und einer Ausgangsvliesbahn, **dadurch gekennzeichnet, dass** die Ausgangsfolienbahn zusammen mit der Ausgangsvliesbahn, deren Schmelzpunkt oberhalb dem Kristallitschmelzpunkt des Polymermaterials liegt, auf eine Temperatur über dem Kristallitschmelzpunkt des Polymermaterials und unter dem Schmelzpunkt der Ausgangsvliesbahn erwärmt und das Laminat durch einen gekühlten Walzenspalt geführt und dabei auf eine Temperatur unterhalb des Kristallitschmelzpunktes der Ausgangsfolienbahn gekühlt wird.

2. Verfahren gemäß Anspruch 1, **dadurch gekennzeichnet, dass** die Ausgangsfolienbahn und die Ausgangsvliesbahn über einen Heizzylinder erwärmt werden, wobei die Temperatur des Heizzylinders so gewählt wird, dass über die Umschlingungsstrecke des Heizzylinders die Ausgangsfolienbahn in den schmelzeflüssigen Zustand erwärmt wird.

3. Verfahren gemäß Anspruch 1 oder 2, **dadurch gekennzeichnet, dass** der gekühlte Walzenspalt von einer Prägewalze und einer Gummiwalze gebildet wird, wobei die Prägestruktur der Prägewalze den Glanzgrad des Laminats reduziert.

4. Verfahren gemäß einem der Ansprüche 1 bis 3, **dadurch gekennzeichnet, dass** das gekühlte Laminat einer Reckung in Maschinen- oder Querrichtung oder in Maschinen- und Querrichtung unterworfen wird.

5. Verfahren gemäß einem der vorhergehenden Ansprüche, **dadurch gekennzeichnet, dass** die Ausgangsfolienbahn blasextrudiert wird.

6. Verfahren gemäß einem der vorhergehenden Ansprüche, **dadurch gekennzeichnet, dass** die Ausgangsfolienbahn bedruckt ist.

7. Verfahren gemäß einem der vorhergehenden Ansprüche, **dadurch gekennzeichnet, dass** die Ausgangsfolienbahn in Maschinen- oder Querrichtung oder in Maschinen- und Querrichtung gereckt ist.

8. Vlies-Folien-Laminate, welche als Sperrschicht für Hygieneartikel verwendbar sind, herstellbar aus einer Ausgangsfolienbahn und einer Ausgangsvliesbahn in einem Verfahren gemäß mindestens einem der Ansprüche 1 bis 7.

9. Laminat gemäß Anspruch 8, **dadurch gekennzeichnet, dass** die Ausgangsfolienbahn aus einer Mischung aus LDPE und LLDPE oder aus einer Mischung aus LDPE, LLDPE und PP besteht.

10. Laminat gemäß Anspruch 8 oder 9, **dadurch gekennzeichnet, dass** die Ausgangsvliesbahn aus PP oder PE besteht.

11. Laminat gemäß einem der Ansprüche 8 bis 10, **dadurch gekennzeichnet, dass** in der Ausgangsvliesbahn und/oder der Ausgangsfolienbahn eine niedrigschmelzende Komponente enthalten ist, wobei die Ausgangsvliesbahn mindestens eine Komponente enthält, die eine Schmelztemperatur oberhalb der Kristallitschmelztemperatur einer bzw. der niedrigschmelzenden Komponente der Ausgangsfolienbahn aufweist.

## Claims

1. A method for the production of nonwoven-film laminates, which are useable as backsheet for personal hygiene articles, from a precursor film web made of thermoplastic polymer and a precursor nonwoven web, **characterized in that** the precursor film web and the precursor nonwoven web, whose melting point is in excess of the crystallite melting point of the polymer, are jointly heated up to a temperature above the crystallite melting point of the polymer and below the melting point of the precursor nonwoven web, and the laminate is guided through a cooled nip, thus being cooled down to a temperature below the crystallite melting point of the precursor film web.

2. The method according to claim 1, **characterized in that** the precursor film web and the precursor nonwoven web are heated through a heating cylinder, wherein the temperature of the heating cylinder is selected such that, over the wrap distance of the heating cylinder, the precursor film web is heated up to the molten state.

3. The method according to claim 1 or 2, **characterized in that** the cooled nip is formed by a stamping roller and a rubber roller, wherein the embossed texture of the stamping roller reduces the degree of gloss of the laminate.

4. The method according to anyone of claims 1 to 3, **characterized in that** the cooled laminate is subjected to a stretching step in machine direction or cross direction or in machine direction and cross direction.

5. The method according to anyone of the preceding claims, **characterized in that** the precursor film web is blow-extruded.

6. The method according to anyone of the preceding claims, **characterized in that** the precursor film web is printed.

7. The method according to anyone of the preceding claims, **characterized in that** the precursor film web is stretched in machine direction or crossways direction or in machine direction and crossways direction.

8. Nonwoven-film laminates, useable as backsheet in personal hygiene articles producible from a precursor film web and a precursor nonwoven web by a method according to at least one of claims 1 to 7.

9. The laminate according to claim 8, **characterized in that** the precursor film web consists of a mixture of LDPE and LLDPE or of a mixture of LDPE, LLDPE and PP.

10. The laminate according to claim 8 or 9, **characterized in that** the precursor nonwoven web consists of PP or PE.

11. The laminate according to anyone of claims 8 to 10, **characterized in that** a low-melting component is contained in the precursor nonwoven web and/or the precursor film web, wherein the precursor nonwoven web contains at least one component which comprises a melting temperature above the crystallite melting temperature of a or the low-melting component of the precursor film web.

## Revendications

1. Procédé pour la préparation de stratifiés de type non-tissé-feuille, qui sont utilisables comme couche de blocage pour articles hygiéniques, à partir d'une bande de feuille de départ en un matériau polymère thermoplastique et d'une bande de non-tissé de départ, **caractérisé en ce que** la bande de feuille de départ est chauffée, ensemble avec la bande de non-tissé de départ, dont le point de fusion est supérieur au point de fusion des cristallites du matériau polymère, à une température supérieure au point de fusion des cristallites du matériau polymère et sous le point de fusion de la bande de non-tissé de départ et le stratifié est guidé à travers une fente de laminage refroidie, tout en étant refroidie à une température inférieure au point de fusion des cristallites de la bande de feuille de départ.

2. Procédé selon la revendication 1, **caractérisé en ce que** la bande de feuille de départ et la bande de non-tissé de départ sont chauffées via un cylindre chauffant, la température du cylindre chauffant étant choisie de manière telle que la bande de feuille de départ est chauffée à l'état de masse fondue liquide sur la zone d'enroulement autour du cylindre chauffant.

3. Procédé selon la revendication 1 ou 2, **caractérisé en ce que** la fente de laminage refroidie est formée par un cylindre gaufreur et un cylindre en caoutchouc, la structure gaufrée du cylindre gaufreur réduisant le degré de brillance du stratifié.

4. Procédé selon l'une quelconque des revendications 1 à 3, **caractérisé en ce que** le stratifié refroidi est soumis à un étirement dans le sens machine ou dans le sens transversal ou dans le sens machine et le sens transversal.

5. Procédé selon l'une quelconque des revendications précédentes, **caractérisé en ce que** la bande de feuille de départ est extrudée-soufflée.

6. Procédé selon l'une quelconque des revendications précédentes, **caractérisé en ce que** la bande de feuille de départ est imprimée.

7. Procédé selon l'une quelconque des revendications précédentes, **caractérisé en ce que** la bande de feuille de départ est étirée dans le sens machine ou dans le sens transversal ou dans le sens machine et le sens transversal.

8. Stratifiés de type non-tissé-feuille, qui sont utilisables comme couche de blocage pour articles hygiéniques, pouvant être préparés à partir d'une bande de feuille de départ et d'une bande de non-tissé de départ dans un procédé selon au moins l'une quelconque des revendications 1 à 7.

9. Stratifié selon la revendication 8, **caractérisé en ce que** la bande de feuille de départ est constituée par un mélange de LDPE et de LLDPE ou par un mélange de LDPE, de LLDPE et de PP.

10. Stratifié selon la revendication 8 ou 9, **caractérisé en ce que** la bande de non-tissé de départ est constituée de PP ou de PE.

11. Stratifié selon l'une quelconque des revendications 8 à 10, **caractérisé en ce que** la bande de non-tissé de départ et/ou la bande de feuille de départ contient un composant à bas point de fusion, la bande de non-tissé de départ contenant au moins un composant qui présente une température de fusion supérieure à la température de fusion des cristallites d'un composant ou, selon le cas, des composants à bas point de fusion de la bande de feuille de départ.
